# EUROPEAN PATENT APPLICATION

(11) **EP 4 817 425 A1**
(43) Date of publication of application: **07.10.2026**
(21) Application number: 24897477.6
(22) Date of filing: 25.11.2024
(51) Int. Cl.: A23L 5/00, A23L 33/17, A61K 9/20

(54) **COMPOSITION AND SOLID MOLDED BODY**

(30) Priority: 29.11.2023 JP 2023202117
(71) Applicant: Nagase & Co., Ltd., Osaka 550-8668 (JP); Nagase Viita Co., Ltd., Okayama-shi, Okayama 702-8006 (JP)
(72) Inventor: TAKAMURA, Haruki, Okayama-shi, Okayama 702-8006 (JP); KOASHI, Yui, Okayama-shi, Okayama 702-8006 (JP); SATO, Takahiro, Tokyo 100-8142 (JP); NAKAZATO, Yukihito, Tokyo 100-8142 (JP); NIWA, Toshiyuki, Nagoya-shi, Aichi 468-8502 (JP)
(74) Representative: Graf von Stosch Patentanwaltsgesellschaft mbH
(86) International application number: PCT/JP2024/041555
(87) International publication number: WO 2025/115792

(57) **Abstract**

An object is to provide a high-protein solid molded body that is excellent in suitability for transport and solubility, and a composition for producing the solid molded body. The object is attained by a composition for producing a solid molded body. The composition contains a protein (a) and a saccharide (b). An amount of the protein (a) is not less than 50% by mass and not more than 90% by mass, and an amount of the saccharide (b) is not less than 10% by mass and not more than 50% by mass, with respect to a total mass of the composition. The saccharide (b) includes maltose.

## Description

### Technical Field

The present invention relates to a composition and a solid molded body.

### Background Art

Conventionally, a technology is known for molding powdered food (for example, protein powder) into a solid form to obtain a solid molded body. For example, Patent Literature 1 discloses a solid milk obtained by compression-molding powdered milk, and Patent Literature 2 discloses a nutrition tablet containing a protein, a carbohydrate, a fat, and tricalcium phosphate.

### Citation List

### [Patent Literature]

[Patent Literature 1] Japanese Patent Application Publication, Tokukai, No. 2022-075913
[Patent Literature 2] Japanese Patent No. 7125978

### Summary of Invention

### Technical Problem

For the solid molded body as described above, it is required to achieve both suitability for transport and solubility. However, with conventional technologies, it has not been possible to achieve both suitability for transport and solubility, particularly in a solid molded body that has a high protein content.

In view of the above circumstances, an object of one aspect of the present invention is to provide a high-protein solid molded body that is excellent in suitability for transport and solubility, and a composition for producing the solid molded body.

### Solution to Problem

In order to attain the object, one aspect of the present invention is a composition for producing a solid molded body, the composition containing a protein (a) and a saccharide (b), wherein an amount of the protein (a) is not less than 50% by mass and not more than 90% by mass, and an amount of the saccharide (b) is not less than 10% by mass and not more than 50% by mass, with respect to a total mass of the composition, and the saccharide (b) includes maltose.

Another aspect of the present invention is a method for producing a solid molded body by molding a composition containing a protein (a) and a saccharide (b), the method including: a tableting step of tableting the composition; a moistening step of moistening a tableted product obtained in the tableting step; and a drying step of drying a moistened tableted product obtained in the moistening step, wherein in the composition, an amount of the protein (a) is not less than 50% by mass and not more than 90% by mass, and an amount of the saccharide (b) is not less than 10% by mass and not more than 50% by mass, with respect to a total mass of the composition, and the saccharide (b) includes maltose.

Still another aspect of the present invention is a method for producing a solid molded body by molding a composition containing a protein (a) and a saccharide (b), the method including: a tableting step of tableting the composition; and a curing step of curing, with use of superheated steam, a tableted product obtained in the tableting step, wherein in the composition, an amount of the protein (a) is not less than 50% by mass and not more than 90% by mass, and an amount of the saccharide (b) is not less than 10% by mass and not more than 50% by mass, with respect to a total mass of the composition, and the saccharide (b) includes maltose.

### Advantageous Effects of Invention

According to one aspect of the present invention, it is possible to provide a high-protein solid molded body that is excellent in suitability for transport and solubility, and a composition for producing the solid molded body.

### Brief Description of Drawings

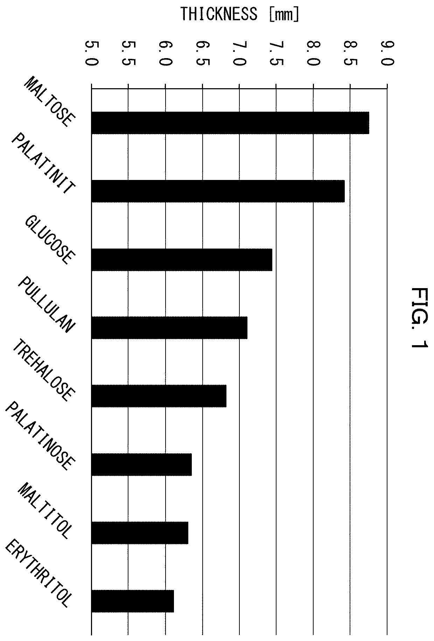

Fig. 1 is a view showing a graph of results of screening of saccharides in Examples.

### Description of Embodiments

Embodiments of the present invention will be described below, but the present invention is not limited thereto. The present invention is not limited to each configuration described below, and various modifications are possible within the scope indicated in the claims. In addition, embodiments or examples obtained by combining technical means respectively disclosed in different embodiments or examples are also included in the technical scope of the present invention. Furthermore, by combining the technical means disclosed in the embodiments, new technical features can be formed. Note that all of the academic literature and patent literature described in the present specification are incorporated herein by reference. In addition, unless otherwise specified in the present specification, any numerical range expressed as "A to B" is intended to mean "not less than A and not more than B".

### [1. Technical idea of the present invention]

Generally, among high-protein foods, those in powder form (commonly referred to as "protein powder" or the like) are widely used. However, such powdered food has a high porosity (approximately 55% to 65%) due to being a powder. In addition, when filling a container with the product, a storage space of approximately 1.88 to 2.1 times the volume of the powder itself is required when the volumes of voids and the volume of a headspace to allow insertion of a measuring spoon are combined, for example, due to the need to secure the headspace and an increase in headspace resulting from compaction during transport. As a result, powdered food have the disadvantage of high storage and transport costs. As society moves toward sustainability, reducing the volume of transported goods and the frequency of transport is an extremely important social issue.

As a means for solving such a problem, a technology has been proposed for molding (solidifying) a powdered food into a solid molded body. If powdered food can be made into a solid molded body, the porosity is significantly reduced, so that storage costs and transport costs can be significantly reduced, which is a very useful solution for creating a sustainable society. In addition, while powdered food generally needs to be stored in a plastic container, the solid molded body can be stored even in a non-plastic container, and thus can also have social value in terms of being plastic-free. Furthermore, since the solid molded body does not need to be measured each time it is used, it can also contribute to an improvement in usability.

On the other hand, as described above, the solid molded body is required to have suitability for transport, that is, fracture resistance such that destruction does not occur due to cracking or crumbling during transport or carrying, and is also required to have solubility to dissolve quickly at the time of use. However, particularly a high-protein food tends to have significantly reduced solubility in water. Therefore, although the solid molded body can have various advantages as compared with the powder form as described above, a high-protein solid molded body that achieves both sufficient suitability for transport and solubility has not been realized with conventional technologies.

In such a situation, the inventors of the present invention conducted diligent studies to provide a high-protein solid molded body that achieves both sufficient suitability for transport and solubility. As a result, they have found that by molding a high-protein powdered composition containing a specific saccharide, it is possible to provide a high-protein solid molded body that achieves both sufficient suitability for transport and solubility, and thus completed the present invention.

A solid molded body that has excellent suitability for transport, excellent solubility, and a high protein content, which are properties that have traditionally been considered conflicting market needs, and a composition capable of providing such a solid molded body have not been conventionally known and can therefore be regarded as a surprising discovery.

### [2. Composition]

A composition in accordance with one embodiment of the present invention (hereinafter, may be referred to as "the present composition") is a composition for producing a solid molded body, the composition containing a protein (a) and a saccharide (b), wherein an amount of the protein (a) is not less than 50% by mass and not more than 90% by mass, and an amount of the saccharide (b) is not less than 10% by mass and not more than 50% by mass, with respect to a total mass of the composition, and the saccharide (b) includes maltose. By molding (solidifying) the present composition by a known method, it is possible to provide a high-protein solid molded body that achieves both sufficient suitability for transport and solubility.

In the present specification, a "high-protein solid molded body" is intended to mean that the solid molded body contains not less than 50% by mass of a protein with respect to a total mass (100% by mass) of the solid molded body.

Hereinafter, the components that can be contained in the present composition will be described in detail.

### (Protein (a))

The present composition contains the protein (a). Hereinafter, "protein (a)" may be referred to as "component (a)". The component (a) can also be said to be a protein composition consisting of one or more types of proteins.

As the protein serving as the component (a), any known protein, preferably a powdered protein, can be used. The protein serving as the component (a) may be an animal protein, may be a plant protein, or may be a combination thereof.

Examples of the animal protein that the present composition can contain as the component (a) include whey protein, casein protein, egg protein, and insect (derived) proteins from crickets, silkworms, and the like. Among them, it is preferable that the present composition contain whey protein as the component (a) because of advantages such as having excellent tableting suitability, having a high-quality flavor, and being easily available.

Examples of the plant protein that the present composition can contain as the component (a) include soy protein, wheat protein, corn protein, buckwheat protein, and proteins derived from seaweed and microalgae.

The content of the component (a) in the present composition is not less than 50% by mass and not more than 90% by mass, preferably not less than 60% by mass and not more than 90% by mass, more preferably not less than 70% by mass and not more than 90% by mass, and still more preferably not less than 80% by mass and not more than 90% by mass, with respect to a total mass (100% by mass) of the present composition. The content (content proportion) of the component (a) in the present composition corresponds to the content (content proportion) of the component (a) in the solid molded body molded from the present composition. Therefore, by setting the content of the component (a) in the present composition within the above range, it is possible to provide a high-protein solid molded body. Note that in a case where the present composition contains two or more types of proteins as the component (a), the content of the component (a) in the present composition means a total amount of the contents of the respective proteins.

### (Saccharide (b))

The present composition contains a saccharide (b). Hereinafter, "saccharide (b)" may be referred to as "component (b)". The component (b) can also be said to be a complex saccharide consisting of one or more types of saccharides.

The content of the component (b) in the present composition is not less than 10% by mass and not more than 50% by mass, preferably not less than 10% by mass and not more than 40% by mass, more preferably not less than 10% by mass and not more than 30% by mass, and still more preferably not less than 10% by mass and not more than 20% by mass, with respect to a total mass (100% by mass) of the present composition. In a case where the present composition contains the component (b) in the above range, it becomes possible to provide a solid molded body that achieves both sufficient suitability for transport and solubility while being a high-protein solid molded body. Note that in a case where the present composition contains two or more types of saccharides as the component (b), the content of the component (b) in the present composition refers to a total amount of the contents of the respective saccharides.

The present composition contains maltose as the saccharide serving as the component (b). The content of the maltose serving as the component (b) in the present composition is not particularly limited, but from the perspective of improving suitability for transport and solubility of an obtained solid molded body, the content is preferably not less than 5% by mass and not more than 30% by mass, more preferably not less than 5% by mass and not more than 20% by mass, and still more preferably not less than 5% by mass and not more than 10% by mass, with respect to a total mass (100% by mass) of the present composition.

The volume-average particle size of the maltose serving as the component (b) in the present composition is not particularly limited, but from the perspective of further improving suitability for transport and solubility of an obtained solid molded body, the volume-average particle size is preferably not less than 10 µm and not more than 350 µm, more preferably not less than 12 µm and not more than 200 µm, and still more preferably not less than 15 µm and not more than 100 µm. Note that in the present specification, the volume-average particle size of the saccharide serving as the component (b) is a value measured by a dry mechanical sieving method (in accordance with Japanese Industrial Standards (JIS) Z8815).

The maltose serving as the component (b) in the present composition may also be porous maltose having a large number of pores. The specific surface area of the porous maltose serving as the component (b) is not particularly limited, but from the perspective of further improving suitability for transport and solubility of an obtained solid molded body, it is preferable that a specific surface area measured by a gas adsorption method using nitrogen gas be not less than 1m²/g.

The pores of the porous maltose serving as the component (b) are not particularly limited, but from the perspective of further improving suitability for transport and solubility of an obtained solid molded body, it is preferable that in a pore distribution measured by mercury intrusion porosimetry, the pores have a pore volume of not less than 0.1 ml/g and show a clear peak at a pore size of less than 5 µm.

The present composition may contain at least maltose as the component (b), but may further contain a saccharide other than maltose. Examples of saccharides other than maltose that the present composition can contain as the component (b) include any monosaccharides, disaccharides, polysaccharides, and sugar alcohols, and examples thereof include trehalose, lactulose, lactose, sucrose, glucose, fructose, galactose, palatinit, pullulan, isomaltulose, isomaltose, maltitol, erythritol, and mannitol. The present composition may contain, as the component (b), only one type or two or more types of these saccharides in addition to maltose.

From the perspective of providing a solid molded body that has even higher solubility, it is preferable that the present composition further contain trehalose as the component (b). The content of trehalose serving as the component (b) in the present composition is not particularly limited, but from the perspective of further improving solubility of an obtained solid molded body, the content is preferably not less than 1% by mass and not more than 20% by mass, more preferably not less than 1% by mass and not more than 15% by mass, and still more preferably not less than 1% by mass and not more than 10% by mass, with respect to a total mass (100% by mass) of the present composition.

The volume-average particle size of trehalose serving as the component (b) in the present composition is not particularly limited, but from the perspective of still further improving solubility of an obtained solid molded body, the volume-average particle size is preferably not less than 10 µm and not more than 600 µm, more preferably not less than 12 µm and not more than 400 µm, and still more preferably not less than 15 µm and not more than 300 µm.

In addition, as trehalose serving as the component (b) in the present composition, porous trehalose can also be used. The specific surface area of the porous trehalose serving as the component (b) is not particularly limited, but from the perspective of further improving suitability for transport and solubility of an obtained solid molded body, it is preferable that a specific surface area measured by a gas adsorption method using nitrogen gas be not less than 1m²/g.

The pores of the porous trehalose serving as the component (b) are not particularly limited, but from the perspective of further improving suitability for transport and solubility of an obtained solid molded body, it is preferable that in a pore distribution measured by mercury intrusion porosimetry, the pores have a pore volume of not less than 0.1 ml/g and show a clear peak at a pore size of less than 5 µm.

From the perspective of providing a solid molded body that has excellent moisture-curing properties, it is preferable that the present composition further contain lactulose as the component (b). The content of lactulose serving as the component (b) in the present composition is not particularly limited, but from the perspective of providing a solid molded body that has excellent moisture-curing properties, the content is preferably not less than 1% by mass and not more than 20% by mass, more preferably not less than 1% by mass and not more than 15% by mass, and still more preferably not less than 1% by mass and not more than 10% by mass, with respect to a total mass (100% by mass) of the present composition.

From the perspective of providing a solid molded body that is excellent in suitability for transport and solubility, as well as moisture-curing properties, it is particularly preferable that the present composition contain, as the component (b), a complex saccharide containing maltose, trehalose, and lactulose.

### (Other component(s))

The present composition may contain another component/other components other than the above-described component (a) and component (b) (may be referred to as "other component(s)") within a range that does not impair the effects of the present invention. Examples of other component(s) that the present composition can contain include, but are not limited to, antifoaming agents, lipids (oils and fats, etc.), amino acids, minerals, vitamins, carbohydrates, lubricants (emulsifiers), and other food additives (for example, flavoring agents, sweeteners, acidulants, colorings, etc.). The present composition may contain only one type of these other components, or may contain two or more types thereof.

In a case where the present composition contains other component(s), the content of the other component(s) in the present composition is not particularly limited as long as the contents of the component (a) and the component (b) are each not less than a predetermined amount, but is preferably not less than 0.01% by mass and not more than 40% by mass, more preferably not less than 0.1% by mass and not more than 30% by mass, and still more preferably not less than 1% by mass and not more than 20% by mass, with respect to a total mass (100% by mass) of the present composition. Note that in a case where the present composition contains a plurality of types of substances as other components, the content of the other components in the present composition is a total content of the plurality of types of other components.

Among the other components described above, the present composition preferably contains an antifoaming agent as another component. This is because an antifoaming agent can suppress the generation of bubbles when the present composition is dissolved in water or the like, which in turn can further improve the solubility of an obtained solid molded body.

As the antifoaming agent that the present composition can contain as another component, various antifoaming agents for food applications can be used, and for example, a (poly)glycerin fatty acid ester or the like can be used.

In a case where the present composition contains an antifoaming agent as another component, the content of the antifoaming agent in the present composition is not particularly limited, but from the perspective of further improving solubility of an obtained solid molded body, it is preferably not less than 0.01% by mass and not more than 40% by mass, more preferably not less than 0.1% by mass and not more than 30% by mass, and still more preferably not less than 1% by mass and not more than 20% by mass, with respect to a total mass (100% by mass) of the present composition. Note that in a case where the present composition contains a plurality of types of substances as other components, the content of the other components in the present composition is a total content of the plurality of types of other components.

### (Others)

The suitability for transport of a solid molded body molded from the present composition is positively correlated with the hardness of the solid molded body after an effect treatment (the higher the hardness after the effect treatment, the better the suitability for transport). The solubility of the solid molded body is positively correlated with the porosity (also referred to as void ratio) of the solid molded body (the higher the porosity, the better the solubility). The porosity of the solid molded body is positively correlated with the thickness of the solid molded body (the larger the thickness, the higher the porosity). Considering this, from the perspective of providing a solid molded body that is even better in both suitability for transport and solubility, it is preferable that a solid molded body formed by curing the present composition satisfy Formula (1) below (1): $y \geq 14.201 {x}^{- 0.071}$
where y represents the thickness (mm) of the solid molded body after a curing treatment, and x represents the hardness (N) of the solid molded body after the curing treatment.

Generally, in a case where tableting is performed with a high load to improve the hardness of a solid molded body, a porous structure in the solid molded body is compressed and destroyed, and the thickness of the solid molded body decreases. The composition satisfying Formula (1) above means that the composition can maintain a sufficient thickness (i.e., a porous structure) even in a case where the composition is tableted under a high load in order to achieve high strength. In other words, this means that the composition is capable of providing a solid molded body that is even better in both suitability for transport and solubility. Note that in the present specification, the hardness (after the curing treatment) of a solid molded body is a value measured by a load cell type tablet hardness tester (for example, PC-30 manufactured by OKADA SEIKO CO., LTD.).

The suitability for transport of a solid molded body molded from the present composition is positively correlated with the hardness of the solid molded body after the curing treatment (the higher the hardness after the curing treatment, the better the suitability for transport). As such, from the perspective of providing a solid molded body that is even better in both suitability for transport and solubility, it is preferable that a solid molded body formed by curing the present composition satisfy Formula (2) below (2): $w \leq 138.29 {e}^{0.0172 v}$
where w represents the dissolution time (seconds) of the solid molded body after the curing treatment, v represents the hardness (N) of the solid molded body after the curing treatment, and e is the base of a natural logarithm (Napier's constant).

Generally, the hardness of a solid molded body has a negative correlation with the dissolution time of the solid molded body, and as the hardness of the solid molded body after the curing treatment increases, the dissolution time of the solid molded body tends to increase (that is, solubility deteriorates). The composition satisfying Formula (2) above means that the composition can be dissolved within a sufficiently short period of time despite having excellent hardness. In other words, this means that the composition is capable of providing a solid molded body that is even better in both suitability for transport and solubility.

The solid molded body in Formulas (1) and (2) above is a solid molded body obtained by tableting 2 g of the present composition to have a diameter of 20 mm and then performing the curing treatment on the tableted composition. As such, for any target composition, when determining whether or not the target composition satisfies Formula (1) and/or Formula (2) above, a solid molded body formed by tableting 2 g of the target composition to have a diameter of 20 mm and then performing the curing treatment on the tableted composition is measured for hardness and/or dissolution time, and the hardness and/or the dissolution time measured is used in the calculation of Formula (1) and/or Formula (2).

Note that the hardness, thickness, and dissolution time of the solid molded body after the curing treatment in Formulas (1) and (2) above can be measured by methods described in Examples. The curing treatment in Formulas (1) and (2) above is intended to refer to operations in a moistening step and a drying step, or an operation in a curing step, all of which will be described later. That is, with respect to Formulas (1) and (2) above, the solid molded body before the curing treatment is intended to mean a tableted product after a tableting step and before the moistening step or the curing step, and the solid molded body after the curing treatment is intended to mean a solid molded body that has undergone the moistening step and the drying step, or the curing step. Therefore, as for specific conditions of each step, conditions described in the section [4. Methods for producing composition and solid molded body] described later can be appropriately adopted.

Note that "hardness" in the above formulas is the hardness of a solid molded body formed by tableting 2 g of the composition to have a diameter of 20 mm and then performing the curing treatment on the tableted composition. For a solid molded body that has a weight and/or a diameter different from the above conditions, it is not preferable to substitute the hardness as it is into the above formulas. Whether or not a solid molded body that has a weight and/or a diameter different from the above conditions satisfies the above formulas can be determined by converting the hardness into a stress. Here, "stress (N/m²) of a solid molded body" means a value obtained by dividing the hardness (N) of the solid molded body by a cross-sectional area of the solid molded body.

### [3. Solid molded body]

In one embodiment of the present invention, a solid molded body molded from the present composition is provided. Hereinafter, "a solid molded body in accordance with one embodiment of the present invention" may be referred to as "the present molded body".

The present molded body is a solid molded body that is molded from the present composition and therefore is excellent in both suitability for transport and solubility while having a high protein content.

### (Composition of the present molded body)

The present molded body is a solid molded body molded from the present composition. As such, components contained in the present molded body and the composition of the components are the same as those of the present composition. Therefore, the present molded body can also be expressed as follows: a solid molded body containing a protein (a) and a saccharide (b), wherein the amount of the protein (a) is not less than 50% by mass and not more than 90% by mass, and the amount of the saccharide (b) is not less than 10% by mass and not more than 50% by mass, with respect to a total mass of the solid molded body, and the saccharide (b) includes maltose.

For the reason described above, for specific aspects of the components contained in the present molded body, the description in the above section [2. Composition] is appropriately incorporated by reference, and the description is omitted in this section.

### (Suitability for transport)

The present molded body is a solid molded body that is excellent in suitability for transport. The suitability of the solid molded body for transport is an index of quality required of the solid molded body during transport. The suitability of the solid molded body for transport can be broadly classified into suitability for transport during production and suitability for transport after the curing treatment. First, one problem that occurs during transport in production is breakage or chipping of the molded body being transferred by a conveyor or a robot hand, during conveyance from the end of tableting to the curing treatment. Such a problem can be solved by improving the hardness (N) of a molded body (tableted product) before the curing treatment. That is, the suitability of the solid molded body for transport during production thereof can be evaluated by the hardness (N) of the molded body (tableted product) before the curing treatment, or by a stress (N/m²) which is a value obtained by dividing the hardness (N) of the solid molded body by a cross-sectional area of the solid molded body. Second, one problem that occurs during transport after the curing treatment is breakage, cracking, or chipping of the solid molded body caused by dropping. Such a problem can be solved by improving the drop strength of the solid molded body after the curing treatment. That is, the suitability of the solid molded body for transport after the curing treatment can be evaluated by the drop strength of the solid molded body.

In the present specification, "a solid molded body that is excellent in suitability for transport" is intended to mean a solid molded body that is excellent in at least the suitability for transport after the curing treatment among the two types of suitability for transport described above. That is, the present molded body is a solid molded body that is excellent in suitability for transport after the curing treatment, and is preferably a solid molded body that is excellent in both suitability for transport during production and suitability for transport after the curing treatment.

Note that in the present specification, the suitability for transport (suitability for transport after the curing treatment) of a solid molded body can be evaluated by a drop strength test by the following methods (1) to (3): (1) five samples of a solid molded body produced under predetermined conditions (specifically, conditions described in Examples) are prepared, and each sample is dropped one by one from a height of 80 cm onto a SUS304 plate having a thickness of 10 mm; (2) the number of times that cracking did not occur in the sample is counted among a total of 5 drops; and (3) a solid molded body for which the number of samples in which cracking did not occur was not less than 3 is evaluated as a solid molded body that is excellent in suitability for transport (suitability for transport after the curing treatment).

### (Solubility)

The present molded body is a solid molded body that is also excellent in solubility. In the present specification, the solubility of a solid molded body can be evaluated by the following method: (1) a sample of a solid molded body produced under predetermined conditions (specifically, conditions described in Examples) is placed in a rotating basket which has an inner diameter of 30 mm and a height of 30 mm and in which holes with a diameter of 2.0 mm are formed at equal intervals on a top surface, a bottom surface, and a wall surface; (2) the rotating basket containing the sample and ion-exchanged water 900 (within an error of 10 ml) are put in a round-bottomed flask, the liquid temperature is maintained at 25.0°C (within an error of 1°C), and the rotating basket containing the sample is rotated at a rotation speed of 300 ± 3 rpm, while the electrical conductivity of the solution (ion-exchanged water) is measured every second; (3) the electrical conductivity at each time is normalized by a value in a state where no sample remains in the rotating basket; and (4) from the normalized result, the dissolution degree of the solid molded body at each time is calculated, and based on a relationship between the calculated dissolution degree and the rotation time, the solubility of the solid molded body is evaluated. Specifically, in the above method, a solid molded body in which the time required for the dissolution degree to reach 95% (for 95% of the solid molded body to dissolve) is shorter than 350 seconds is evaluated as a solid molded body having excellent solubility.

Note that the solid molded body being excellent in solubility (that is, satisfying the above criteria) means that the solid molded body is not only easily dissolved in a liquid at a relatively high temperature (85°C) such as for dissolving powdered milk, but also is quickly dissolved in various liquids at a relatively low temperature (approximately 5°C to 40°C) in which the solid molded body is generally considered difficult to dissolve, such as water and various liquids using water as a solvent (particularly, milk, soft drinks, coffee, etc.). In other words, the solid molded body being excellent in solubility means that the solid molded body is suitable for daily use (ingestion).

### (Applications of solid molded body)

The present molded body can be suitably used as food intended for daily consumption, more specifically, for applications such as luxury food items including confections, health foods, health supplement foods, foods with health claims, foods for specified health uses, foods with nutrient function claims, supplements, or foods with function claims.

### [4. Methods for producing composition and solid molded body]

### <Method for producing the present composition>

The method for producing the present composition is not particularly limited, and the present composition can be produced by weighing and mixing not less than 50 parts by mass and not more than 90 parts by mass of the component (a), not less than 10 parts by mass and not more than 50 parts by mass of the component (b), and other optional components in accordance with a known method so as to achieve a total amount of 100 parts by mass.

The amounts of the component (a), the component (b), and the other optional components used in the method for producing the present composition correspond to the respective contents in the solid molded body obtained. Therefore, in the method for producing the present composition, it is preferable to adjust the amounts of the respective components used so that the resulting solid molded body has a desired composition, particularly a desired protein content.

### <Method for producing the present molded body>

The method for producing the present molded body is not particularly limited, provided that the present molded body can be obtained by molding the present composition. However, from the perspective of obtaining a solid molded body that has an even better suitability for transport, suitable methods include a method that includes a tableting step, a moistening step, and a drying step, or a method that includes a tableting step and a curing step. That is, it is preferable that the method for producing the present molded body be one of the following methods:
a method for producing a solid molded body by molding a composition containing the protein (a) and the saccharide (b), the method including a tableting step of tableting the composition, a moistening step of moistening a tableted product obtained in the tableting step, and a drying step of drying a moistened tableted product obtained in the moistening step, wherein in the composition (i.e., the present composition), the amount of the protein (a) is not less than 50% by mass and not more than 90% by mass, and the amount of the saccharide (b) is not less than 10% by mass and not more than 50% by mass, with respect to a total mass of the composition, and the saccharide (b) includes maltose (Method 1); and
a method for producing a solid molded body by molding a composition containing the protein (a) and the saccharide (b), the method including a tableting step of tableting the composition, and a curing step of curing, with use of superheated steam, a tableted product obtained in the tableting step, wherein in the composition, the amount of the protein (a) is not less than 50% by mass and not more than 90% by mass, and the amount of the saccharide (b) is not less than 10% by mass and not more than 50% by mass, with respect to a total mass of the composition, and the saccharide (b) includes maltose (Method 2).

### (Method 1)

First, a preferable aspect of the method for producing the present molded body will be described in detail by taking a method including a tableting step, a moistening step, and a drying step (Method (1)) as an example. Note that for a composition containing predetermined amounts of the protein (a) and the saccharide (b) used in the present production method (that is, the present composition), the description in the above section [2. Composition] is incorporated by reference, and the description is omitted in this section.

### (Tableting step)

It is preferable that the method for producing the present molded body include a tableting step of tableting the present composition. By performing the tableting step, it is possible to provide a solid molded body that is even better in hardness, and it is also possible to suppress deformation of a molded product (tableted product) made of the present composition in a subsequent moistening step and drying step.

In the tableting step, the method of tableting the present composition is not particularly limited, but it is preferable to perform tableting using a tableting machine because the tableting force (also referred to as the tableting load) and the size of the obtained tableted product are easy to control, and also for reasons of productivity.

The tableting force in the tableting step is preferably such that the resulting tableted product has a hardness sufficient to suppress breakage or chipping of the tableted product during conveyance to the subsequent moistening step and drying step, and to suppress deformation of the tableted product in the subsequent moistening step and drying step. Specifically, the tableting force in the tableting step is not particularly limited, provided that the resultant tableted product has a hardness of not less than 3 N and not more than 10 N, and preferably not less than 3 N and not more than 5 N. The upper limit of the tableting force is not particularly limited, but can be not more than 6 kN. Note that the hardness of the tableted product can also be regarded as a hardness of the present molded body before the curing treatment.

The thickness of the tableted product obtained in the tableting step is correlated with the porosity of the tableted product. This means that a tablet obtained by tableting an equivalent amount of the composition exhibits higher porosity with increasing thickness. Therefore, from the perspective of providing a solid molded body having a high porosity, it is preferable to tablet the present composition in the tableting step such that the resultant tableted product has as large a thickness as possible. However, in a case where a tableting force is reduced to increase the thickness of the obtained tableted product, the strength of the obtained tableted product also decreases. That is, in conventional technology, the thickness and the hardness of the obtained tableted product are in a trade-off relationship, and it has been difficult to obtain a tableted product that achieves both thickness and hardness. The present composition, however, contains the component (b). As such, the present composition can provide a tableted product having a sufficient hardness of not less than 10 N even in a case where the present composition is tableted at a relatively weak tableting force (for example, approximately 3 kN to 6 kN) to increase the thickness of the obtained tableted product. Therefore, it is possible to provide a tableted product having a large thickness, in other words, a high porosity, and consequently to provide a solid molded body having a high porosity and excellent solubility.

### (Moistening step)

It is preferable that the method for producing the present molded body include a moistening step of moistening the tableted product obtained in the tableting step to obtain a moistened tableted product. When the moistening step is performed, some of the particles of the present composition on the surface of the tableted product become liquid or gel-like and cross-link with each other. As a result, a strong structure made of the present composition can be formed on the surface of the moistened tableted product. As a result, the strength of an obtained solid molded body can be improved, and a solid molded body that is even more excellent in suitability for transport can be provided.

In the moistening step, the method for moistening the tableted product is not particularly limited, and examples thereof include a method of leaving the tableted product to stand in a high-humidity environment, a method of directly spraying water or the like onto the tableted product, and a method of blowing steam onto the tableted product. The method of moistening is not particularly limited, and any method such as a continuous method or a batch method may be used.

In a case where the method of leaving the tableted product to stand in a high-humidity environment is adopted, the humidity of the environment is not particularly limited, but can be, for example, not less than 60% RH and not more than 100% RH, preferably not less than 80% RH and not more than 100% RH, and more preferably not less than 90% RH and not more than 100% RH.

In the moistening step, the longer the time for moistening the tableted product (moistening time), the greater the hardness tends to be for the resultant moistened tableted product and, consequently, for the obtained solid molded body. As such, from the perspective of hardness, the longer the moistening time, the more preferable it is. However, the longer the moistening time, the lower the production efficiency of the solid molded body. As such, from the perspective of productivity, the shorter the moistening time, the more preferable it is. Therefore, from the perspective of achieving both excellent hardness and productivity, the moistening time in the moistening step is preferably not less than 10 seconds and not more than 300 seconds, more preferably not less than 20 seconds and not more than 250 seconds, and still more preferably not less than 30 seconds and not more than 200 seconds.

### (Drying step)

It is preferable that the method for producing the present molded body include a drying step in which the moistened tableted product obtained in the moistening step is dried to obtain a solid molded body. By performing the drying step, stickiness (tack) can be eliminated from the liquid or gel-like structure formed on the surface of the moistened tableted product in the moistening step, and as a result, it is possible to provide a solid molded body that not only has a strong structure formed of the inter-crosslinked present composition but also is excellent in handleability. This series of operations consisting of the moistening step and the drying step may be referred to as a moistening curing step.

In the drying step, the method of drying the moistened tableted product is not particularly limited, and examples thereof include a method of leaving the moistened tableted product to stand in a low-humidity and high-temperature environment, a method of drying the moistened tableted product using a dryer, and a method of blowing hot air onto the moistened tableted product (hot-air drying).

In the drying step, the temperature for drying the moistened tableted product (drying temperature) is not particularly limited, but can be, for example, 20°C to 90°C, preferably 30°C to 80°C, and more preferably 40°C to 60°C.

### (Method 2)

Next, another preferable aspect of the method for producing the present molded body will be described in detail by taking a method including a tableting step and a curing step (Method (2)) as an example. Note that with respect to Method (2), specific aspects of the tableting step are the same as those of the tableting step of (Method (1)), and therefore the description is omitted in this section.

### (Curing step)

It is preferable that the method for producing the present molded body include a curing step of curing, with use of superheated steam, a tableted product obtained in the tableting step. The curing step can also be regarded as a step of bringing the tableted product into contact with superheated steam. By performing the curing step, a strong structure made of the present composition can be formed on the surface of the tableted product, similarly to the moistening step described above, and at the same time, stickiness (tack) can be eliminated from the liquid or gel-like structure formed on the surface of the tableted product, similarly to the drying step described above. As a result, it is possible to provide a solid molded body that not only has a strong structure formed of the inter-crosslinked present composition but also is excellent in handleability. In other words, the curing method using superheated steam can be regarded as a method that performs moistening and drying at the same time. Note that superheated steam is intended to mean water vapor heated to at least the boiling point of water.

In the curing step, the method of treating the tableted product with superheated steam is not particularly limited, and examples thereof include a method of introducing the tableted product into a constant temperature and humidity chamber, and a method of spraying superheated steam onto the tableted product in a conveyor-type continuous furnace.

The temperature of the superheated steam used in the curing step is not particularly limited, but is preferably 100°C to 300°C, more preferably 110°C to 250°C, and still more preferably 120°C to 220°C.

In the curing step, the longer the time for contacting the tableted product with superheated steam (curing time), the greater the hardness of the obtained solid molded body tends to be. As such, from the perspective of hardness, the longer the curing time, the more preferable it is. On the other hand, the longer the curing time, the lower the production efficiency of the solid molded body. As such, from the perspective of productivity, the shorter the curing time, the more preferable it is. As such, from the perspective of achieving both excellent hardness and productivity, the curing time in the curing step is preferably not less than 10 seconds and not more than 300 seconds, more preferably not less than 20 seconds and not more than 250 seconds, and still more preferably not less than 30 seconds and not more than 200 seconds.

In the method for producing the present molded body, the hardness of a solid molded body obtained through the moistening step and the drying step, or through the curing step (hardness of the present molded body after the curing treatment) is preferably not less than 10 N, more preferably not less than 15 N, and still more preferably not less than 25 N, from the perspective of providing a solid molded body that is even more excellent in suitability for transport. From the perspective of solubility, the hardness of the solid molded body is preferably not more than 30 N. That is, from the perspective of achieving both suitability for transport and solubility, the hardness of the solid molded body is preferably 10 N to 30 N, more preferably 20 N to 30 N, and still more preferably 25 N to 30 N.

### [5. Others]

One aspect of the present invention may include the following features.
<1> A composition for producing a solid molded body, the composition containing a protein (a) and a saccharide (b), wherein an amount of the protein (a) is not less than 50% by mass and not more than 90% by mass, and an amount of the saccharide (b) is not less than 10% by mass and not more than 50% by mass, with respect to a total mass of the composition, and the saccharide (b) includes maltose.
<2> The composition described in <1>, wherein the saccharide (b) further includes trehalose.
<3> The composition described in <1>, wherein the maltose is contained in an amount of not less than 5% by mass and not more than 30% by mass with respect to the total mass of the composition.
<4> The composition described in <2>, wherein the trehalose is contained in an amount of not less than 1% by mass and not more than 20% by mass with respect to the total mass of the composition.
<5> The composition described in any one of <1> to <4>, wherein the saccharide (b) further includes lactulose.
<6> The composition described in any one of <1> to <5>, wherein the protein (a) includes whey protein.
<7> The composition described in any one of <1> to <6>, further containing an antifoaming agent.
<8> The composition described in any one of <1> to <7>, wherein a solid molded body molded from the composition satisfies Formula (1) below (1): $y \geq 14.201 {x}^{- 0.071}$ where y represents a thickness (mm) of the solid molded body after a curing treatment, and x represents a hardness (N) of the solid molded body after the curing treatment.
<9> The composition described in any one of <1> to <8>, wherein a solid molded body molded from the composition satisfies Formula (2) below (2): $w \leq 138.29 {e}^{0.0172 v}$ where w represents a dissolution time (seconds) of the solid molded body after a curing treatment, and v represents a hardness (N) of the solid molded body after the curing treatment.
<10> A solid molded body, molded from a composition described in any one of <1> to <9>.
<11> A method for producing a solid molded body by molding a composition containing a protein (a) and a saccharide (b), the method including: a tableting step of tableting the composition; a moistening step of moistening a tableted product obtained in the tableting step; and a drying step of drying a moistened tableted product obtained in the moistening step, wherein in the composition, an amount of the protein (a) is not less than 50% by mass and not more than 90% by mass, and an amount of the saccharide (b) is not less than 10% by mass and not more than 50% by mass, with respect to a total mass of the composition, and the saccharide (b) includes maltose.
<12> A method for producing a solid molded body by molding a composition containing a protein (a) and a saccharide (b), the method including: a tableting step of tableting the composition; and a curing step of curing, with use of superheated steam, a tableted product obtained in the tableting step, wherein in the composition, an amount of the protein (a) is not less than 50% by mass and not more than 90% by mass, and an amount of the saccharide (b) is not less than 10% by mass and not more than 50% by mass, with respect to a total mass of the composition, and the saccharide (b) includes maltose.

### Examples

Next, the present invention will be described in more detail based on Examples, but the present invention is not limited only to such Examples.

### [Experimental Example 1: screening test of saccharides]

### (Test method)

The tableting characteristics of various saccharides were confirmed, and saccharides suitable for the production of a solid molded body were screened.

First, 2.0 g each (within an error of 0.003 g) of a protein powder (WPC392 manufactured by Fonterra Japan Limited) and any one of the saccharides shown below were weighed and mixed to prepare a composition containing 50 parts by weight each of the protein and the saccharide (weight ratio 1:1).

### · Saccharides used

Maltose, palatinit, glucose, pullulan, trehalose, palatinose, maltitol, and erythritol

Each of the obtained compositions was tableted with the tableting force appropriately adjusted so that the composition would have a hardness of 5 N after being tableted. As a result, a tableted product having a hardness of 5 N was obtained. The thickness of the obtained tableted product was measured. The results are shown in Fig. 1. Note that in the Examples, the thicknesses of a tableted product and a solid molded body are values each measured using a caliper (ABS digimatic caliper CD-AX, manufactured by Mitutoyo Corporation), and the hardnesses of a tableted product and a solid molded body are values each measured using a load cell type tablet hardness tester (PC-30 manufactured by OKADA SEIKO CO., LTD.).

### (Results)

When tableting was performed so as to achieve a hardness of the tableted product of 5 N, the saccharide that exhibited the largest thickness of the tableted product was maltose. Since the thickness of the tableted product is correlated with the solubility of the final solid molded body, it was suggested that maltose is the most suitable saccharide for providing a solid molded body that is excellent in both suitability for transport and solubility.

### [Examples 1 and 2 and Comparative Examples 1 and 2]

### (Preparation of compositions)

Materials of the types and ratios shown in Table 1 were mixed to obtain powdered compositions. Details of the materials used are as follows.

### · Materials

### Component (a)

a-1: Protein powder mainly containing whey protein (manufactured by FONTERRA JAPAN LIMITED, product name "WPC392")

### Component (b)

b-1: Maltose (manufactured by KABUSHIKIKAISYA HAYASHIBARA, product name "SUNMALT (registered trademark) Midori")
b-2: Trehalose (manufactured by KABUSHIKIKAISYA HAYASHIBARA, product name "TREHA (registered trademark)")
b-3: Lactulose (manufactured by MORINAGA MILK INDUSTRY CO., LTD., product name "Milk Oligosaccharide MLC (registered trademark)-97")

### Antifoaming agent

Antifoaming agent preparation (manufactured by Riken Vitamin Co., Ltd., product name "Poem ZL-3")

### Other saccharides

### Lactose

Note that a commercially available protein powder may contain a trace amount of impurities in addition to protein, but in the present specification, the amount (content) of the protein powder used, inclusive of any impurities, is regarded as the content of the component (a) in the composition.

### [Table 1]

**(Table 1)**

| | | Example 1 | Example 2 | Comparative Example 1 | Comparative Example 2 |
|---|---|---|---|---|---|
| Material | | Blending amount (parts by weight) | | | |
| Component (a) | a-1 | 87.5 | 50.0 | 87.5 | 50.0 |
| Component (b) | b-1 | 7.0 | 28.0 | | |
| | b-2 | 3.0 | 12.0 | | |
| | b-3 | 2.0 | 8.0 | | |
| Other components | Defoaming agent | 0.5 | 2.0 | | |
| | Lactose | | | 12.5 | 50.0 |
| Total | | 100 | 100 | 100 | 100 |

The obtained compositions and molded bodies molded from the respective compositions were measured or evaluated for tableting characteristics, moistening curing characteristics, suitability for transport, and solubility in accordance with the following methods.

### (Tableting characteristics)

2.000 g of each of the compositions was weighed out, and tableted into a cylindrical shape with a diameter of 2.0 cm at a tableting force indicated in Table 2 using a tableting machine, to obtain a tableted product. The obtained tableted product was measured for hardness and thickness. The results are shown in Table 2.

### [Table 2]

**(Table 2)**

| | | Example 1 | Example 2 | Comparative Example 1 | Comparative Example 2 |
|---|---|---|---|---|---|
| Tableting force: 1 kN | Hardness [N] | 2.77 | 5.44 | 0.59 | 1.35 |
| | Thickness [mm] | 14.64 | 13.73 | 14.65 | 12.47 |
| Tableting force: 2 kN | Hardness [N] | 6.20 | 10.65 | 1.76 | 2.40 |
| | Thickness [mm] | 13.68 | 12.94 | 13.72 | 11.73 |
| Tableting force: 3 kN | Hardness [N] | 10.50 | 17.58 | 3.48 | 4.55 |
| | Thickness [mm] | 12.84 | 12.34 | 13.20 | 11.22 |
| Tableting force: 4 kN | Hardness [N] | 15.63 | 25.78 | 4.41 | 6.17 |
| | Thickness [mm] | 12.59 | 11.95 | 12.75 | 10.98 |
| Tableting force: 5 kN | Hardness [N] | 22.04 | 30.03 | 6.62 | 9.12 |
| | Thickness [mm] | 12.19 | 11.55 | 12.29 | 10.88 |

As is clear from Table 2, tableted products formed by tableting compositions 1 and 2, each of which satisfies the requirements specified for the present composition, are superior in hardness to tableted products formed by tableting comparative compositions 1 and 2, when tableted at the same tableting force. In addition, for example, a comparison between the results for the composition 1 tableted at 2 kN and the comparative composition 1 tableted at 5 kN indicates that, when providing tableted products similar in hardness, a tableted product having a greater thickness can be provided by using a composition that satisfies the requirements specified for the present composition. That is, it was shown that by tableting the present composition, it is possible to provide a tableted product that is excellent in thickness and hardness and can provide a solid molded body that is excellent in suitability for transport and solubility.

### (Moistening curing characteristics)

By a method described in the above section "(Tableting characteristics)", each of the above compositions was tableted at a tableting force adjusted to achieve a hardness of 1.00 N. Thus, a tableted product having a hardness of 1.00 N was obtained. The obtained tableted product was left to stand for moistening in a thermostatic chamber at a humidity of 95% RH and a temperature of 80°C for each time (moistening time) indicated in Table 3, to obtain a moistened tableted product. The obtained moistened tableted product was measured for hardness. The results are shown in Table 3. Note that as shown in Table 3, some of the moistened tableted products derived from the comparative composition 1 and the moistened tableted products derived from the comparative composition 2 had not been cured at the end of a predetermined moistening time, and could not be measured for hardness.

### [Table 3]

**(Table 3)**

| Moistening time | | Composition 1 | Composition 2 | Comparative Composition 1 | Comparative Composition 2 |
|---|---|---|---|---|---|
| Moistening time: 60 s | Hardness [N] | 3.47 | 25.07 | - *1 | - *1 |
| Moistening time: 90 s | Hardness [N] | 5.00 | 34.90 | - *1 | - *1 |
| Moistening time: 120 s | Hardness [N] | 8.38 | 42.74 | - *1 | - *1 |
| Moistening time: 180 s | Hardness [N] | 20.38 | 58.62 | - *1 | 1.86 |
| Moistening time: 240 s | Hardness [N] | 29.40 | > 100.00 | - *1 | 2.28 |

| | | | | | |
|---|---|---|---|---|---|
| *1 Unmeasurable because hardness measurement could not be performed. | | | | | |

As is clear from Table 3, moistened tableted products derived from the compositions 1 and 2, each of which satisfy the requirements specified for the present composition, can achieve a remarkably high hardness in a shorter time, as compared with moistened tableted products derived from the comparative compositions 1 and 2. That is, it was shown that by further moistening a tableted product formed by tableting the present composition, it is possible to efficiently provide a moistened tableted product that is excellent in hardness and therefore can provide a solid molded body excellent in suitability for transport.

### (Suitability for transport (drop strength))

3 g (within an error of 0.001 g) of each of the above compositions was tableted using a tableting machine at a tableting force adjusted to achieve a hardness of the composition of 1.0 N. Thus, a tableted product having a hardness of 1.0 N was obtained (tableting step). Next, the obtained tableted product was left to stand for moistening in a thermostatic chamber at a humidity of 95% RH and a temperature of 80°C for 240 seconds, to obtain a moistened tableted product (moistening step). Subsequently, the obtained moistened tableted product was placed in a dryer at a temperature of 60°C and dried for 10 minutes, to obtain a solid molded body (drying step).

The suitability for transport of each of the obtained solid molded bodies was evaluated by the following methods: (1) for each solid molded body, five samples were prepared, and each sample was dropped one by one from a height of 80 cm onto a SUS304 plate having a thickness of 10 mm; (2) for each solid molded body, the number of times that cracking did not occur in the sample was counted among a total of 5 drops; and (3) for each molded body, based on the number of samples in which cracking did not occur, the suitability for transport of the molded body was evaluated according to the following criteria. Evaluation results of the suitability for transport are shown in Table 4.

### · Evaluation criteria for suitability for transport

Number of samples in which cracking did not occur is not less than 3: excellent in suitability for transport (excellent) Number of samples in which cracking did not occur is not more than 2: poor in suitability for transport (poor).

### [Table 4]

**(Table 4)**

| | Composition 1 | Composition 2 | Comparative Composition 1 | Comparative Composition 2 |
|---|---|---|---|---|
| Number of times cracking occurred | 2 | 0 | 5 | 5 |
| Evaluation of suitability for transport | Excellent | Excellent | Poor | Poor |

As is clear from Table 4, solid molded bodies molded from the comparative compositions 1 and 2 have a poor suitability for transport, whereas solid molded bodies molded from the compositions 1 and 2, each of which satisfies the requirements specified for the present composition, have an excellent suitability for transport. That is, it was shown that by molding the present composition, it is possible to provide a solid molded body that is excellent in suitability for transport.

### (Solubility)

3 g (within an error of 0.001 g) of each of the above compositions was tableted using a tableting machine at a tableting force adjusted to achieve a hardness of the composition of 1.0 N. Thus, a tableted product having a hardness of 1.0 N was obtained (tableting step). Next, the obtained tableted product was left to stand for moistening in a thermostatic chamber at a humidity of 95% RH and a temperature of 80°C for 240 seconds, to obtain a moistened tableted product (moistening step). Subsequently, the obtained moistened tableted product was placed in a dryer at a temperature of 60°C and dried for 10 minutes, to obtain a solid molded body with a hardness of 10 N or 20 N (drying step).

The solubility of each obtained solid molded body was evaluated by the following method: (1) a sample of the produced solid molded body was placed in a rotating basket which had an inner diameter of 30 mm and a height of 30 mm and in which holes with a diameter of 2.0 mm were formed at equal intervals on a top surface, a bottom surface, and a wall surface; (2) the rotating basket containing the sample and 900 mL (within an error of 10 mL) of ion-exchanged water were put in a round-bottomed flask, the liquid temperature was maintained at 25.0°C (within an error of 1°C), and the rotating basket containing the sample was rotated at a rotation speed of 300 ± 3 rpm, while the electrical conductivity of the solution (ion-exchanged water) was measured every second; (3) the electrical conductivity at each time was normalized by a value in a state where no sample remained in the rotating basket; and (4) from the normalized result, the dissolution degree of the solid molded body at each time was calculated, and based on a relationship between the calculated dissolution degree and the rotation time, the solubility of the solid molded body was evaluated according to the following criteria. Evaluation results of the solubility are shown in Table 5. Note here that normalization of the electrical conductivity was performed according to the following formula. 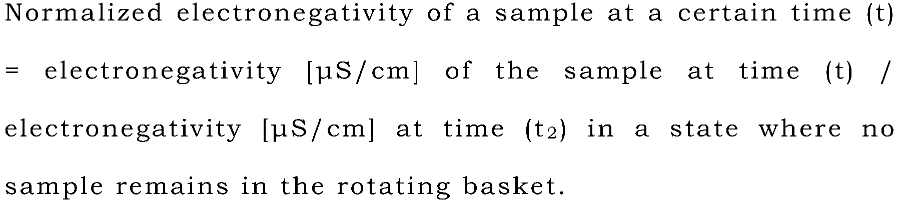 Normalized electronegativity of a sample at a certain time (t) = electronegativity [µS/cm] of the sample at time (t) / electronegativity [µS/cm] at time (t2) in a state where no sample remains in the rotating basket.

### · Evaluation criteria for solubility

Time required for solubility to reach 95% (for 95% of the solid molded body to dissolve) is shorter than 350 seconds: excellent in solubility (excellent)
Time required for solubility to reach 95% (for 95% of the solid molded body to dissolve) is not less than 350 seconds: poor in solubility (poor)
Solubility after 500 seconds of rotation is less than 5%: insoluble ((the solid molded body hardly or does not dissolve at all in water) (insoluble)).

Note that in the present specification, "dissolution time (after a curing treatment) of a solid molded body" is a time required for 100% of a solid molded body, which is formed by tableting and curing 2 g of a composition to have a diameter of 20 mm in the same procedure as the operations described above, to dissolve when the operations of the above (1) to (4) are performed on the solid molded body.

### [Table 5]

**(Table 5)**

| | | Composition 1 | Composition 2 | Comparative Composition 1 | Comparative Composition 2 |
|---|---|---|---|---|---|
| Hardness: 10 N | Solubility evaluation | Excellent | Excellent | Insoluble | Poor |
| Hardness: 20 N | | Excellent | Excellent | Insoluble | Insoluble |

As is clear from Table 5, solid molded bodies molded from the comparative composition 1 and a solid molded body molded to a hardness of 20 N from the comparative composition 2 were not dissolved in water at all. A solid molded body molded from the comparative composition 2 to a hardness of 10 N was dissolved in water but showed only poor solubility. In contrast, it can be seen that solid molded bodies molded from the compositions 1 and 2, which satisfied the requirements specified for the present composition, were excellent in solubility. That is, it was shown that by molding the present composition, it is possible to provide a solid molded body that is excellent in solubility.

From the above results, it was shown that a solid molded body molded from the present composition is a high-protein solid molded body that is excellent in suitability for transport and solubility.

### Industrial Applicability

A solid molded body in accordance with one embodiment of the present invention can be suitably used in the food field and the like as a solid molded body that not only has a high protein content but also is excellent in suitability for transport and solubility.

## Claims

1. A composition for producing a solid molded body, the composition comprising a protein (a) and a saccharide (b), wherein:
an amount of the protein (a) is not less than 50% by mass and not more than 90% by mass, and an amount of the saccharide (b) is not less than 10% by mass and not more than 50% by mass, with respect to a total mass of the composition; and
the saccharide (b) includes maltose.

2. The composition as set forth in claim 1, wherein the saccharide (b) further includes trehalose.

3. The composition as set forth in claim 1, wherein the maltose is contained in an amount of not less than 5% by mass and not more than 30% by mass with respect to the total mass of the composition.

4. The composition as set forth in claim 2, wherein the trehalose is contained in an amount of not less than 1% by mass and not more than 20% by mass with respect to the total mass of the composition.

5. The composition as set forth in claim 1, wherein the saccharide (b) further includes lactulose.

6. The composition as set forth in claim 1, wherein the protein (a) includes whey protein.

7. The composition as set forth in claim 1, further comprising an antifoaming agent.

8. The composition as set forth in claim 1, wherein a solid molded body molded from the composition satisfies Formula (1) below (1): $y \geq 14.201 {x}^{- 0.071}$ where y represents a thickness (mm) of the solid molded body after a curing treatment, and x represents a hardness (N) of the solid molded body after the curing treatment.

9. The composition as set forth in claim 1, wherein a solid molded body molded from the composition satisfies Formula (2) below (2): $w \leq 138.29 {e}^{0.0172 v}$ where w represents a dissolution time (seconds) of the solid molded body after a curing treatment, and v represents a hardness (N) of the solid molded body after the curing treatment.

10. A solid molded body, molded from a composition recited in any one of claims 1 to 9.

11. A method for producing a solid molded body by molding a composition containing a protein (a) and a saccharide (b), the method comprising:
a tableting step of tableting the composition;
a moistening step of moistening a tableted product obtained in the tableting step; and
a drying step of drying a moistened tableted product obtained in the moistening step,
wherein in the composition,
an amount of the protein (a) is not less than 50% by mass and not more than 90% by mass, and an amount of the saccharide (b) is not less than 10% by mass and not more than 50% by mass, with respect to a total mass of the composition, and
the saccharide (b) includes maltose.

12. A method for producing a solid molded body by molding a composition containing a protein (a) and a saccharide (b), the method comprising:
a tableting step of tableting the composition; and
a curing step of curing, with use of superheated steam, a tableted product obtained in the tableting step,
wherein in the composition,
an amount of the protein (a) is not less than 50% by mass and not more than 90% by mass, and an amount of the saccharide (b) is not less than 10% by mass and not more than 50% by mass, with respect to a total mass of the composition, and
the saccharide (b) includes maltose.
